# EUROPÄISCHE PATENTSCHRIFT

(11) **EP 2 015 804 B1**
(45) Veröffentlichungstag und Bekanntmachung des Hinweises auf die Patenterteilung: **13.02.2019**
(21) Anmeldenummer: 07720101.0
(22) Anmeldetag: 27.04.2007
(51) Int. Cl.: A61M 5/142, A61M 5/162, A61M 5/172, A61M 5/168, A61M 5/14

(54) **INFUSIONSSET MIT EINER DATENSPEICHERVORRICHTUNG**
INFUSION APPARATUS WITH A DATA STORAGE DEVICE
ENSEMBLE DE PERFUSION DOTÉ D'UN DISPOSITIF D'ENREGISTREMENT DE DONNÉES

(30) Priorität: 10.05.2006 CH 7512006
(43) Veröffentlichungstag der Anmeldung: 21.01.2009
(73) Patentinhaber: F. Hoffmann-La Roche AG, 4070 Basel (CH); Roche Diabetes Care GmbH, 68305 Mannheim (DE)
(72) Erfinder: WOCHNER, Micaela, 8127 Forch (CH); LINIGER, Jürg, 3072 Ostermundigen (CH); WYSS, Martin, 3400 Burgdorf (CH)
(74) Vertreter: Rentsch Partner AG
(86) Internationale Anmeldenummer: PCT/CH2007/000204
(87) Internationale Veröffentlichungsnummer: WO 2007/128144

(56) Entgegenhaltungen:
- WO-A-01/85232
- US-A- 5 647 854
- US-A- 5 739 508
- US-A1- 2004 193 453
- US-A1- 2005 277 890

## Beschreibung

Die vorliegende Erfindung betrifft ein Infusionsset zu einer extrakorporal getragenen Vorrichtung zur kontinuierlichen Verabreichung eines medizinischen oder pharmazeutischen Wirkstoffes in ein organisches Gewebe.

Derartige Vorrichtungen, beispielsweise bestehend aus einem Infusionsgerät und einem Infusionsset, werden in der ambulanten Insulinpumpentherapie zur Stabilisierung der Blutzuckerwerte bei Diabetespatienten eingesetzt.

Ein gebräuchliches Infusionsset besteht aus einem infusionsgeräteseitigen Konnektor welcher über einen Katheterschlauch mit dem Katheterkopf fluidisch verbunden ist. Am Katheterkopf ist eine Kanüle angebracht welche vom Patienten in das subkutane Körpergewebe eingeführt wird.

Die kontinuierliche Wirkstoffverabreichung mittels Infusionsgerät und Infusionsset stellt eine Reihe grundsätzlicher Anforderungen damit der therapeutische Erfolg sichergestellt werden kann. Aus hygienischen Gründen, zur Vermeidung einer Insulinresistenz und um allergischen Reaktionen vorzubeugen muss ein Infusionsset nach ungefähr 2-3 Verwendungstagen mit wechselnder Lokalisation ausgetauscht werden und darf nur einmal verwendet werden. Geeignete, sogenannte autorisierte, Infusionssets werden von den Herstellern von Infusionsgeräten oder durch autorisierte Hersteller angeboten und in sterilen Verpackungen abgegeben.

Der Austausch des Infusionssets wird vom Insulinpumpenträger eigenhändig vorgenommen. Ebenso obliegt es dem Patienten sicherzustellen, dass wiederum ein autorisiertes, nicht überaltertes und ungebrauchtes Infusionssets zur Anwendung kommt. Im Handel sind jedoch eine Vielzahl Infusionssets erhältlich welche standardgemässe Luer-Lock-Anschlüsse aufweisen und somit grundsätzlich die Konnektion zwischen Infusionspumpe und Infusionsset ermöglichen, jedoch nicht für den Betrieb mit einem bestimmten Infusionsgerät geeignet sind bzw. vom Hersteller nicht autorisiert sind.

Die kontinuierliche Wirkstoffverabreichung mittels Infusionsgerät und Infusionsset stellt eine Reihe zusätzlicher Anforderungen, damit der therapeutische Erfolg sichergestellt werden kann bzw. das von einem therapiekonformen Infusionsset gesprochen werden kann.

Für ein therapiegemässes Infusionsset wird vorausgesetzt, dass vor Gebrauch des Infusionssets und vor Einbringen der Kanüle in das Körpergewebe die Fluidleitung vollständig und luftblasenfrei mit Wirkstoff befüllt ist. Dieser Vorgang wird allgemein als "Primingvorgang oder Priming" bezeichnet. Aufgrund der Modellvielfalt der Infusionssets mit jeweils unterschiedlichen Schlauchlängen resp. Füllvolumen muss der Primingvorgang vom Patienten manuell vorgenommen und überwacht werden. Ein Priming gilt als erfolgreich abgeschlossen, wenn an der Spitze der noch nicht eingeführten Kanüle ein Tropfen des zu verabreichenden Medikamentes austritt. Eine visuelle Überwachung des Tropfenaustrittes setzt hohe Anforderungen an das Sehvermögen des Diabetespatienten was oft, aufgrund des diabetestypischen Krankhietsprofiles, zu einer Überforderung führt. Wird durch ein so genanntes "Überpriming" zu viel Flüssigkeit gefördert besteht die Gefahr einer unkontrollierten Kontamination der Umgebung mit Wirkstoff.

Aufgrund des Routinecharakters können solche vom Pumpenträger zu bewältigenden Aufgaben vermehrt zu sicherheitskritische Situationen führen.

Im Stand der Technik sind Infusionssets mit integriertem Datenspeicher bekannt welche einen Schutz vor Verwendung unautorisierter oder bereits verwenderten Infusionsset über Identifikationssystemen gewährleisten. Beispielsweise offenbart EP 1132102 A2 eine medizintechnische Vorrichtung mit einem an einem Verabreichungsgerät angeschlossenen Zubehörteil, welches verschlüsselte oder unverschlüsselte Informationen bezüglich des bisherigen Gebrauches aufweist, womit eine Mehrfachverwendung verhindert wird.

US2005/00599926 A1 beschreibt eine fluidische Zuführung als Teil eines Infusionssets mit einem Datenspeicher welcher die individuellen Fliesswiderstands- und Strömungsbeiwerte des betreffenden Infusionssets beinhaltet um eine genauere Verabreichung des fluidischen Wirkstoffes zu ermöglichen.

Ein Infusionsset mit einem RFID-Datenspeicher welcher die vom Arzt verordneten Verabreichungsdaten enthält wird in US2004/0193453 A1 offenbart. Damit wird sichergestellt, dass das richtige Medikament in der richtigen Dosierung verabreicht wird.

US5739508 offenbart ein Verfahren zur Speicherung und zum Auslesen von Informationen bei Verwendung eines Elementes zur Administration medizinischer Flüssigkeiten. Dabei wird in einem Speicher des Elementes, insbesondere eines medizinischen Flüssigkeitsbehälters, vom Hersteller oder Lieferanten Information gespeichert, die anschliessend am Ort der Verwendung ausgelesen und zur Berechnung geeigneter Betriebsparameter einer Vorrichtung zur Flüssigkeitsverabreichung verwendet wird.

US2005/0107923 A1 beschreibt eine Vorrichtung welche aufgrund der, in einer fluidischen Zuführung z. B. einer Katheterleitung, festgestellten Druckverhältnisse ermittelt ob bei mehrfacher Katheterzuführung der richtige Katheter mit dem richtigen Medikamentenreservoir verbunden ist.

Der Erfindung liegt die Aufgabe zugrunde, die im Stand der Technik geschilderten Nachteile beim Austausch und beim Betrieb einer mit einem Infusionsset zusammenwirkenden Infusionsvorrichtung zu überwinden und sicherzustellen, dass Abweichungen zum therapiekonformen Betriebszustand eines an einer Verabrei-chungsvorrichtung angeschlossenen Infusionssets automatisiert oder unter Mithilfe des Pumpenträgers erkannt und behoben werden können. Dies wird mit einer Vorrichtung gemäss Anspruch 1 und einem Verfahren gemäss Anspruch 16 erreicht.

Die Erfindung geht von einem Infusionsset mit einer dazu vorgesehenen Verabreichungsvorrichtung aus. Das erfindungsgemässe Infusionsset basiert in seiner fluidischen Ausgestaltung auf Ausführungsformen welche im Stand der Technik bekannt sind. Die Konnektion zwischen Infusionsset und einer dazu vorgesehenen Infusionsvorrichtung besteht aus einer Luer-Lock-Verbindung welche in Form eines Gewindeverschlusses schliesst und öffnet. Aufgabengleiche proprietäre Konnektoren sind ebenfalls im Stand der Technik bekannt.

Eine allgemeine Ausführungsform einer Verabreichungsvorrichtung stellt eine Insulinpumpe für die kontinuierliche Abgabe von medizinischen Wirkstoffen mit einem gekoppelten Infusionsset dar. Eine vom Arzt verordnete Insulinmenge wird mittels der Insulinpumpe zur therapiegemässen Stabilisierung des Blutzuckerspiegels ausgeschüttet. Die Insulinpumpe kann aufgrund der kompakten Bauweise diskret und körpernah getragen werden. Die räumliche Distanz zwischen optimaler Trageposition der Insulinpumpe und Injektionsort wird mit der flexiblen Katheterleitung des Infusionssets überwunden. Eine mit dem erfinderischen Infusionsset kooperierend Verabreichungsvorrichtung weist eine prozessorgesteuerte Dosiervorrichtung auf mit welcher die Wirkstoffdosierung abgegeben wird. Die Art und Weise der verabreichungsgemässen Dosierung ist dem Fachmann bekannt und muss hier nicht erläutert werden. Ebenfalls sind in Stand der Technik Insulinpumpen bekannt, welche über Daten- oder Kommandointerfaces mit externen Geräten in Verbindung treten können.

Wird zunächst aufgrund der Datenlage auf dem Datenspeicher des Infusionssets festgestellt, dass ein bezüglich der Verabreichungsvorrichtung autorisiertes Infusionsset vorliegt, werden geeignete Instruktionen der Verabreichungsvorrichtung zugeführt. Falls keine autorisierte Verabreichungsvorrichtung vorliegt, wird eine Verwendung mit der Verabreichungsvorrichtung verhindert oder entsprechend eingeschränkt.

Ein weiterer Vorteil der mit der geschaffenen Interaktion zwischen Infusionsset und Verabreichungsvorrichtung ergibt sich aus der Möglichkeit Informationen bezüglich sicherheitskritischen Betriebszustände, welche nachfolgend als "nicht therapiekonform" zusammengefasst werden, zu übermitteln. Solche den therapeutischen Erfolg gefährdende Betriebszustände sind:
- ein Leck im Fluidkanal des Infusionssets liegt vor;
- eine Okklusion im Fluidkanal des Infusionsset tritt auf, z.B. wenn der Katheterschlauch durch aufliegende Körperteile zusammengequetscht wird;
- die Konnektion zwischen Infusionsset und Verabreichungsvorrichtung wurdenicht richtig vorgenommen;
- die Konnektion zwischen zwischen Head und Tube des Katheterkopfes wurde nicht richtig vorgenommen wurde;
- das Infusionsset sitzt nicht richtig auf dem Körper.

Auf dem erfinderischen Infusionsset befindet sich ein Datenspeicher mit geeigneten Daten welcher über eine Datenaustauschvorrichtung datenprozessierend mit einer zur Verwendung vorgesehenen Verabreichungsvorrichtung in Verbindung steht. Geeignete Daten auf dem Datenspeicher können Informationen wie etwa "Infusionsset Typ" aus welchen sich ein Primingvolumen für den Katheterschlauch ableiten lässt, "Katheterschlauchlänge" oder "Füllvolumen für den Primingvorgang" sein.

Als Datenspeicher können grundsätzlich überschreibbare und nicht-überschreibbare Informationsträger, wie sie in ihren Hauptausführungsarten als RAM- oder ROM-Speicher allgemein bekannt sind zur Anwendung kommen. In einer einfachsten Ausführungsform als fest verdrahteter ROM-Logikspeicher oder mittels manuell konfigurierbaren DIP-Schaltern als RAM-Speicher. ROM Datenspeicher enthalten in der erfinderischen Ausführungsform herstellerseitig vorgegebene Informationen wie beispielsweise Seriennummer, Verfallsdatum und Schlauchlänge, Füllvolumen des Infusion-Sets. Eine spezielle Ausführungsform stellt der WORM-Datenspeicher (Write-Once-Read-Many times) dar, welche ein einmaliges Beschreiben des Datenträgers mit Informationen erlaubt. Nach einem erstmalig ausgeführten Schreibvorgang kann nur noch gelesen werden.

RAM Speicher sind in der erfinderischen Ausführungsform geeignet alarm- und betriebszustandsbezogene Informationen wie "Auftreten einer Okklusion", "Auftreten eines Lecks", "Infusion-Set ist zur Pumpe nicht richtig angeschlossen" oder beispielsweise "Infusion-Set zwischen Katheterkopf und Katheterschlauch nicht richtig angeschlossen" abgespeichert zu haben und mit den aktualisierten Werten nachgeführt zu werden.

In einer bevorzugten Ausführungsform befinden sich aufgabenspezifische Sensoren auf dem Infusionsset, welche ausgelegt sind spezifische Betriebszustände zu erfassen und als resultatsgemäss als elektrisches Ausgangssignal zur Verfügung zu stellen. Solche Detektoren sind als Okklusions-, Konnektions- oder Leckdetektoren dem Fachmann bekannt. Im Gegensatz zu einem Sensor stellt ein Detektor bereits die aufgabenspezifischen Zustandsinformationen in binärer Form zur Verfügung. So stellt beispielsweise ein Okklusionsdetektor seine Outputinformationen in Wahr/Falsch-Form dar. Der Zustand "Wahr" korrespondiert dabei mit der Aussage "Auftreten einer Okklusion" oder umgekehrt.

Um die Informationen des Datenspeichers der Verabreichungsvorrichtung, beispielsweise einer Insulinpumpe, zuzuführen, ist eine Datenaustauschvorrichtung vorgesehen.

Im Stand der Technik sind zahlreiche Möglichkeiten bekannt um eine Datenaustauschvorrichtunge zu realisieren. Zum Beispiel mittels drahtloser Übermittlungstechnik mit Übermittlungsprotokollen welche verschlüsselte oder unverschlüsselte Datenübertragungsmodi erlauben.

In einer besonders bevorzugten Form werden Datenspeicherung und der Datenaustausch mittels RFID (Radio Frequency Identification) Technologie vorgenommen welche auch in Nahbereichsidentifikationssystemen zur Anwendung kommen.

Eine weitere bevorzugte Ausführungsform basiert auf optisch basierten Übertragungs- oder Identifikationssystemen, wie beispielsweise Strichcodeslesesysteme oder Weiterentwicklungen wie etwa 2D-Strichcode- oder Hologramm-Systeme.

Einige Infusionssets erlauben dem Benutzer eine temporäre Dekonnektierung des Katheterschlauches vom Katheterkopf damit der Patient die Pumpe, beispielsweise während eines Bades oder einer Dusche, vom Körper entfernen kann. In einer besonders bevorzugten Ausführungsweise wird dafür eine Aufteilung der Datenspeicher in zwei autonome Datenspeicher/Transponder vorgenommen. Je eine Datenspeicher/Transpondereinheit befindet sich auf dem proximalen und auf dem distalen Abschnitt eines Infusionssets. Damit kann auch ermittelt werden ob die konnektierbaren Einheiten noch immer in der initialen Konfiguration vorliegen.

In einer weiteren bevorzugten Ausführungsform beruht die Datenübertragung auf einem optischen Identifikationsverfahren. Ein Lesegerät, beispielsweise ein in der Infusionsvorrichtung integrierter Strichcodescanner, erlaubt bedarfsweise ein Auslesen der Informationen vom Infusionsset. Der Strichcode übernimmt dabei die Rolle eines optischen ROM Speichers.

Wird ein optisches Übertragungsverfahren angewendet, befindet sich der Datenspeicher vorzugsweise auf dem distalen Katheterkopfabschnitt, dem proprietären oder Luer-Lock-Konnektor, einer am Infusionsset angebrachten Banderole oder auf der Infusionssetverpackung.

Eine mögliche Ausführungsform stellt die drahtgebundene Datenübertragung dar. Elektrische Leitungen und entsprechende Kontaktverbindungen am Konnektor übertragen die notwendigen Signale die zum Datenaustausch notwendig sind. Durch die generell äusserst geringen Signalströme wird der Energieverbrauch der versorgenden Verabreichungsvorrichtung nur unwesentlich beansprucht. Die auf dem Infusionsset als fest verdrahteten Speichereinheiten oder DIP-Schalter vorliegenden Datenspeicher sind dabei galvanisch mit der Kontrolleinheit verbunden.

In einer rein mechanischen Ausführungsform bilden mechanische Kodiernocken im Konnektor ebenfalls eine Speicherausführungsform.

Die geschaffene Interaktivität zwischen Verabreichungsvorrichtung und Infusionsset erlaubt die Anwendung zahlreicher Verfahren, welche geeignet sind die Therapie- und Patientensicherheit zu erhöhen und die Bedienungsfreundlichkeit zu steigern. Ein erster massgebender Schritt stellt die Identifikation ob ein autorisiertes Infusionsset vorliegt dar. Nach erfolgreicher Identifikation können die die Fähigkeiten der Verabreichungsvorrichtung verändert oder erweitert werden oder spezifische Abläufe veranlassen. Die Resultate aller Identifikationsvorgänge können auf der Verabreichungsvorrichtung protokolliert werden und stehen Rückverfolgbarkeitsanalysen zur Verfügung.

Ein bevorzugtes Verfahren zur Erweiterung oder zur Initiierung von Fähigkeiten der Infusionsvorrichtung stellt das durch die Erfindung erstmals ermöglichte "Autopriming" dar. In diesem besonders bevorzugten Verfahren werden Datenspeicherinformationen des Infusionssets welche ein Primingvolumen definieren oder bestimmbar machen bei Bedarf, d.h. nach einer erstmaligen Konnektierung eines neuen Infusionssets, ausgelesen. Liegt ein als gültig anerkanntes Resultat vor, kann beispielsweise nach erfolgter Befehlsquittierung durch den Patienten ein automatisierter Primingvorgang auf der Infusionsvorrichtung ausgelöst werden.

Das massgebliche Flüssigkeitsvolumen für ein Priming, welches durch die Katheterlänge und dem Durchflussfläche des Katheterschlauches definiert ist, ist entwender direkt als Volumen oder über eine Konstante, im Sinne der Datenverarbeitung, im Datenspeicher abrufbar.

In einem besonders bevorzugten Verfahren kann das Primingvolumen aus den Typenbezeichnungsinformationen des Infusionssets bestimmt werden. Die typenspezifischen Primingvolumina können auf der Insulinpumpe referenziert oder in einer sogenannten "look-up table" auf der Verabreichungsvorrichtung abgelegt sein.

In einem ebenfalls bevorzugten Verfahren, mit höchsten Anforderungen bezüglich Primingvolumengenauigkeit, wird das erforderliche Primingvolumen vom Hersteller individualtypisch bestimmt und als Parameter auf dem Infusionsset abgespeichert.

In einer weiteren bevorzugten Ausführungsform überwacht ein, auf dem Infusionsset in unmittelbarer Nähe des Kanülenaustrittes befindlicher Primingdetektor das Befüllen des Infusionssets. Wird ein Primingvorgang vom Pumpenträger veranlasst detektiert der Primingsensor unter Echtzeitbedingungen das Erreichen des Medikamentfluids an der Sensorposition. Unter Berücksichtigung des Restvolumens zwischen Sensorposition und Kanülenauslass wird der Primingvorgang abgeschlossen. Das Ausgangssignal des Primingsensors wird über den Datenspeicher und Datenaustauschvorrichtung der Kontrolleinheit als Rückmeldung zugeführt.Ein Leitfähigkeitssensor im fluidischen Kanülenabschnitt mit einer definierten Schwellenwertcharakteristik stellt eine einfache Realisierungsmöglichkeit eines sogenannten Primingdetektors dar.

In einem weiteren Verfahren wird bei Erreichen des Tragedauerablaufes des Infusionssets der Datenspeicher von der Infusionsvorrichtung derart beeinflusst, das eine Weiterverwendung verunmöglicht ist. In einer bevorzugten Ausführungsform wird die entsprechende Information in einen WORM-Datenspeicher abgelegt welcher nach einem erfolgten Schreibvorgang keine Statusänderung mehr zulässt. Dadurch wird das Infusionsset gewissermassen softwaremässig entwertet.

Ein weiteres bevorzugtes Verfahren stellt die Erkennung einer fehlerhaften Konnektion zwischen Verabreichungsvorrichtung und Infusionsset dar. Bei einer einwandfreien Konnektion wird eine galvanische oder mechanische Verbindung hergestellt oder unterbrochen welche eine geeignete Zustandsinformation darstellt. Falls ein nicht einwandfrei konnektiertes Infusionsset erkannt wird, erscheint eine sinngemässe Hinweismeldung auf der Infusionsvorrichtung.

Im Folgenden werden bevorzugte Ausführungsformen der Erfindung anhand einiger Figuren näher erläutert.

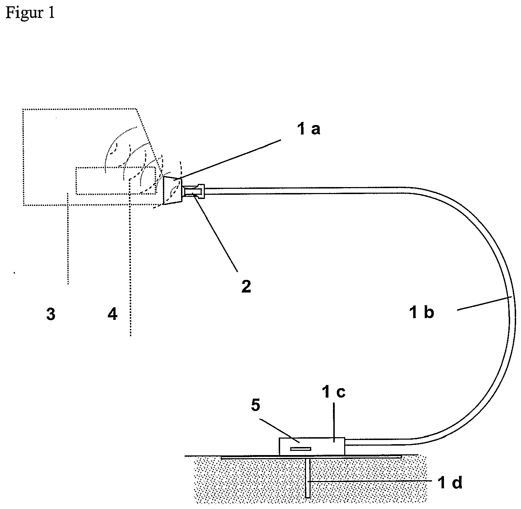

Figur 1 zeigt ein Infusionsset (1a,1b,1c) mit einer in das Körpergewebe eingebrachten Kanüle (1d) welches mit einer Verabreichungsvorrichtung (3) konnektiert ist. Der Datenspeicher (2) befindet in der Nähe des fluidischen Konnektors (1a) und ist mit einem RFID Close-Coupling-System mit der Lese- und Kontrolleinrichtung (4) verbunden. Bei Close-Coupling-Systemen beträgt die Reichweite ungefähr 1 cm. Die Datenübertragung kann aufgrund der kurzen Distanz über eine induktive oder kapazitive Kopplung zwischen Lesegerät und Transponder vorgenommen werden. Aufgrund niedriger Signalpegeln und guten Abschirmmöglichkeiten des Signalweges kann ein hoher Sicherheitsstandard gewährleistet werden. Ein weiterer Vorteil der starren Anordnung zwischen Lesegerät und Transponder besteht darin, dass ein konstanter Signalpegel herrscht. Durch die geringe Übermittlungsdistanz mit niedrigen Signalpegeln ist der Energiebedarf der Transponder/Datenspeichereinrichtung auf dem Infusionsset gering und kann dem Koppel- oder Übertragungsfeld entnommen werden. Auf dem Katheterkopf (1c) befindet sich ein oder mehrere aufgabenspezifische Sensoren (5), beispielsweise ein Okklusionsdetektor, ein Leckdetektor oder ein Primingdetektor welcher datenprozessierend mit dem Datenspeicher (2) verbunden ist und seine aktuelle Statusinformation dem Speicher überträgt. Die Datenübertragung zum Datenspeicher (2) erfolgt über eine galvanische Verbindung welche im Katheterschlauch integriert ist. Die Spannungsversorgung, welche wahlweise über elektrische Kontakte am Konnektor oder über eine Entnahme aus dem Koppelfeld erfolgt, wird über über eine im Katheterschlauch vorhandene Kabelverbindung dem Sensor auf dem Katheterkopf zugeführt.

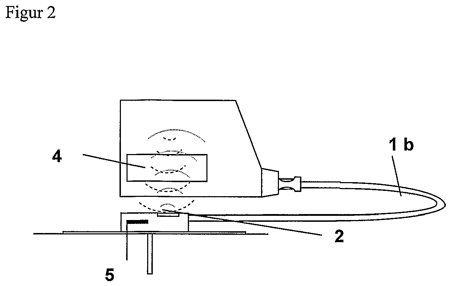

Figur 2 zeigt eine Dosiereinrichtung zur Verabreichung einer medizinischen Substanz (3) mit einem konnektierten Infusionsset (1a,1b,1c) gemäss einer weiteren Ausführungsform unter Verwendung eines Close Coupling RFID Systems mit einer Speicher/Transpondereinheit (2) welche sich aus Platzgründen im Katheterkopf befindet. Soll der gewünschte Identifikationsvorgang zwischen Infusionsset und Infusionsvorrichtung ermöglicht werden, muss die Lesevorrichtung auf der Verabreichungsvorrichtung (4) zum Katheterkopf mit dem integrierten RFID-Tag geführt und positioniert werden. Soll ein automatisierter Primingvorgang durchgeführt werden werden, muss zuerst eine Identifikation durchgeführt werden ob ein Infusionsset mit geeigneten Daten, welche ein Primingvolumen beschreiben oder ableitbar machen, angeschlossen ist. Wird aufgrund einer Validitätsprüfung festgestellt, dass die Dateninformationen ein sicheres Primen erlauben, wird bei noch nicht in das Körpergewebe eingebrachter Kanüle, der Katheterschlauch mit dem Wirkstoff befüllt. Ein am Katheterkopf angebrachter Prminsensor übermittelt das Zustanssignal über den RAM-Datenspeicher welches einen erfolgreichen Primingvorgang anzeigt.

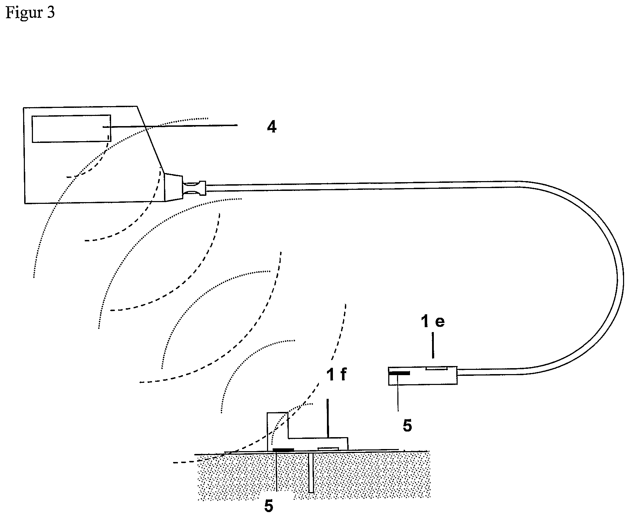

Figur 3 zeigt ein Infusionsset welches dem Pumpenträger eine kurzzeitige Dekonnektierung des Katheterschlauches vom Katheterkopf erlaubt, um beispielsweise Pumpe und Katheterschlauch vor einem Bad oder zu Duschzwecken abnehmen zu können. Die fluidische Trennung erfolgt am Katheterkopf wobei der proximale Teil des Katheterkopfes(1f) auf der Körperoberfläche verbleibt und einen RFID-Tag oder Transponder/Datenspeicher aufweist. Diese Ausführungsform erlaubt eine Identifikation des proximalen Teils des Infusionssets auch bei nicht konnektiertem Katheterschlauch. In der dargestellten Ausführungsform wird ein Remote-Coupling RFID System verwendet welches eine Reichweite bis zu 1 m ermöglicht. Der distale Teil des Katheterkopfes (1e) bleibt mit dem Katheterschlauch (1b) an der Verabreichungsvorrichtung verbunden (1b). Diese Ausführungsform erlaubt eine Identifikation des Infusionssets auch bei nicht konnektiertem Katheterschlauch. Zusätzlich besteht mit dieser Ausführungsform eine Möglichkeit festzustellen falls Katheterschlauch und Katheterkopf nicht mehr in einer therapiekonformen Konfiguration z. B längst verfallenes Katheterschlauchteil (Tube-Set) und neuem distalen Katheterkopfteil (Head-Set), vorliegen. Ebenfalls ist ein Nachprimen mir dem Primingvolumen des distalen Katheterkopfteiles (Head-Set) möglich, wenn das Head-Set (5a) vorzeitig ausgetauscht worden ist.

## Patentansprüche

1. Infusionsset zur Verabreichung einer medizinischen Flüssigkeit, umfassend:
- einen Datenspeicher (2) zum Austausch von Daten über eine Datenaustauschvorrichtung (4) mit einer Verabreichungsvorrichtung (3), wobei die auf dem Datenspeicher (2) vorliegenden Informationen über eine Datenaustauschvorrichtung (4) der Verabreichungsvorrichtung (3) zur Feststellung von Abweichungen zum therapiekonformen Betriebszustand übermittelt werden, damit automatisiert oder unter Mithilfe des eines Pumpenträgers ein therapiekonformer Betriebszustand herbeigeführt werden kann, wobei der Datenspeicher (2) mindestens einen überschreibbaren Datenbereich aufweist, wobei die Daten auf den überschreibbaren Datenbereichen von der autorisierten Verabreichungsvorrichtung (3) beeinflusst werden können,
- mindestens einen auf dem Infusionsset lokalisierten Zustandsdetektor (5), wobei die Daten auf den überschreibbaren Datenbereichen von einem Ausgangssignal des mindestens einen auf dem Infusionsset lokalisierten Zustandsdetektors (5) geändert werden können.

2. Infusionsset nach Anspruch 1, **dadurch gekennzeichnet, dass** der auf dem Infusionsset lokalisierte Zustandsdetektor (5) ein Okklusions-, ein Leck-, ein Konnektierungs- oder ein Primingsensor sein kann.

3. Infusionsset nach Anspruch 2, **dadurch gekennzeichnet, dass** das Ausgangssignal eines Zustandsdetektors als Statusflag im überschreibbaren Datenbereichen abgespeichert wird.

4. Infusionsset nach Anspruch 1, **dadurch gekennzeichnet, dass** auf dem Datenspeicher (2) Speicherbereiche (WORM) vorhanden sind, welche von der Infusionsvorrichtung über die Datenaustauschvorrichtung genau einmal geändert werden können, um beispielsweise eine Kennzeichnung bzw. Entwertung eines dauerhaft sicherheitskritischen Infusionssets vornehmen zu können.

5. Infusionsset nach Anspruch 4, **dadurch gekennzeichnet, dass** der WORM- Datenspeicher (2) nach Ablauf der Tragedauer von der Infusionsvorrichtung über die Datenaustauschvorrichtung im Sinne einer Entwertung verändert wird, sodass eine erneute Verwendung verunmöglicht wird.

6. Infusionsset nach Anspruch 1, **dadurch gekennzeichnet, dass** der Datenspeicher (2) mindestens einen nicht-überschreibbaren (ROM) Datenbereich aufweist, der mindestens eine typenspezifische Kennung enthält, welche ein spezifisches Primingvolumen definiert oder bestimmbar macht, damit gegebenenfalls ein automatisierter oder halbautomatisierter Primingvorgang veranlasst werden kann, sodass ein therapiekonformes Infusionsset vorliegt.

7. Infusionsset nach einem der vorangehenden Ansprüche, **dadurch gekennzeichnet, dass** die Datenaustauschvorrichtung (4) auf drahtloser Übermittlungstechnik beruht.

8. Infusionsset nach Anspruch 7, **dadurch gekennzeichnet, dass** die Datenaustauschvorrichtung (4) auf drahtloser RFID-Technologie beruht.

9. Infusionsset nach Anspruch 8, **dadurch gekennzeichnet, dass** sich je eine RFID- Speicher/Transpondereinheitauf dem proximalen Teil (1f) des Katheterkopfes und/oder auf dem übrigen Katheterabschnitt (1e, 1b, 1a) befindet.

10. Infusionsset nach einem der Ansprüche 1 bis 6, **dadurch gekennzeichnet, dass** der auf dem Infusionsset angebrachte Datenspeicher (2) über eine galvanische Verbindung am Konnektor mit der Verabreichungs-vorrichtung (3) in Verbindung steht.

11. Infusionsset nach den Ansprüchen 1 bis 5, **dadurch gekennzeichnet, dass** der auf dem Infusionsset (1) angebrachten Datenspeicher (2) mit einem optischen Übertragungsverfahren, wie beispielsweise IrDA, mit der Datenaustauschvorrichtung (4) mit der Verabreichungsvorrichtung (3) in Verbindung steht.

12. Infusionsset nach Anspruch 6, **dadurch gekennzeichnet, dass** der auf dem Infusionsset angebrachte Datenspeicher (2) über ein unidirektionales optisches Verfahren, wie beispielsweise Strichcodeidentifikationsverfahren, mit der Verabreichungsvorrichtung (3) in Verbindung steht.

13. Infusionsset nach Anspruch 6, **dadurch gekennzeichnet, dass** der auf dem Infusionsset angebrachten Datenspeicher über mechanische Codiernocken am Konnektor mit der Verabreichungsvorrichtung (3) in Verbindung steht.

14. System bestehend aus einem Infusionsset zur Verabreichung einer medizinischen Flüssigkeit gemäss Anspruch 1 und einer dazu vorgesehener Verabreichungsvorrichtung (3).

15. Verfahren zur Interaktion eines Infusionssets (1) gemäss Anspruch 1 mit einer Verabreichungsvorrichtung (3), **dadurch gekennzeichnet, dass** bei Vorliegen von Datenspeicherinformationen, aus welchen sich ableiten lässt, dass eine nicht geeignete bzw. nicht therapiekonforme Konfiguration eines Infusionssets mit einer Verabreichungsvorrichtung vorliegt, eine Warnmeldung auf der Verabreichungsvorrichtung ausgelöst wird und/oder eine Verwendung des Infusionssets verhindert wird.

16. Verfahren nach Anspruch 15, **dadurch gekennzeichnet, dass** ein von einem Primingdetektor gesteuerter, automatischer oder semi-automatischer Primingvorgang ausgeführt wird, falls zur Schaffung eines therapiekonformen Betriebszustandes ein Primingvorgang notwendig ist und falls Datenspeicherinformationen in Echtzeit vorliegen, welche den Signalzustand des Primingdetektors wiedergeben.

17. Verfahren nach den Anspruch 15 **dadurch gekennzeichnet, dass** bei Vorliegen von Daten auf dem Datenspeicher (2), welche ein Primingvolumen eines Infusionsset definieren oder bestimmbar machen, ein automatischer oder semi-automatischer Primingvorgang auf der Verabreichungsvorrichtung (3) ermöglicht werden kann.

18. Verfahren nach Anspruch 15, **dadurch gekennzeichnet, dass** nach der Überschreitung der Tragedauer eines an einer Verabreichungsvorrichtung angeschlossenen Infusionssets von der Infusionsvorrichtung (3) über die Datenaustauschvorrichtung (4) entwertet bzw. auf dem Datenspeicher (2) gekennzeichnet wird, womit eine erneute Verwendung verunmöglicht wird.

19. Verfahren nach den Ansprüchen 15 und 18, **dadurch gekennzeichnet, dass** bei Vorliegen von Datenspeicherinformationen, aus welchen festgestellt oder abgeleitet werden kann, dass ein bereits verwendetes oder entwertetes Infusionsset erneut konnektiert ist, der Benutzer aufgefordert wird, den Gebrauch des Infusionssets zu unterlassen und/oder eine weitere Verwendung verhindert wird.

20. Verfahren nach den Ansprüchen 15 und 19, **dadurch gekennzeichnet, dass** bei Vorliegen von Datenspeicherinformationen, aus welchen festgestellt werden kann, dass ein angeschlossener Konnektierungdetektor eine fehlerhafte Konnektierung zwischen Infusionsset und Verabreichungsvorrichtung feststellt, der Benutzer aufgefordert wird, den Mangel zu beheben und eine Verwendung bis zur Mangelbehebung verhindert wird.

21. Verfahren nach den Ansprüchen 15, 19 und 20, **dadurch gekennzeichnet, dass** bei Vorliegen von Datenspeicherinformationen, aus welchen festgestellt werden kann, dass ein angeschlossener Okklusionsdetektor eine Okklusion im Infusionsset feststellt, der Benutzer aufgefordert wird, die Okklusion zu beheben.

22. Verfahren nach den Ansprüchen 15, 19, 20 und 21, **dadurch gekennzeichnet, dass** bei Vorliegen von Datenspeicherinformationen, aus welchen festgestellt werden kann, dass ein angeschlossener Leckdetektor ein Leck im Infusionsset feststellt, der Benutzer aufgefordert wird, die Ursachen des Lecks zu beheben.

## Claims

1. Infusion set for administering a medical fluid, comprising:
- a data memory (2) for exchanging data via a data exchange device (4) with an administration device (3) wherein the information present in the data memory (2) is transmitted via a data exchanges device (4) of the administration device (3) in order to determine deviations from the treatment-conforming operating status so that in an automated manner or via assistance by the pump wearer a treatment-conforming operating status can be effected, wherein the data memory (2) has at least one overwritable data area, wherein the data in the overwritable data areas can be influenced by the authorised administration device (3),
- at least one status detector (5) located on the infusion set, wherein the data on the overwritable data areas can be amended by an output signal of the at least one status detector (5) located on the infusion set.

2. Infusion set according to claim 1 **characterised in that** the status detector (5) located on the infusion set can be an occlusion, a leakage, a connection or a priming sensor.

3. Infusion set according to claim 2 **characterised in that** the output signal of a status detector is stored as a status flag in the overwritable data areas.

4. Infusion set according to claim 1 **characterised in that** in the data memory (2) storage areas (WORM) are present which via the data exchange device can be changed precisely once by the infusion device in order, for example, to identify and/or invalidate a continuously safety-critical infusion set.

5. Infusion set according to claim 4 **characterised in that** the WORM data memory (2) on expiry of the usage duration of the infusion device is amended in the form of an invalidation via the data exchange device so that renewed use is made impossible.

6. Infusion set according to claim 1 **characterised in that** the data memory (2) has at least one non-overwritable (ROM) data area which contains at least one type-specific identifier which defines or makes determinable a specific priming volume so that, if necessary, an automated or semi-automated priming process can be brought about so that a treatment-conforming infusion set is available.

7. Infusion set according to any one of the preceding claims **characterised in that** the data exchange device (4) is based on wireless transmission technology.

8. Infusion set according to claim 7 **characterised in that** the data exchange device (4) is based on wireless RFID technology.

9. Infusion set according to claim 8 **characterised in that** an RFID memory/transponder unit is present on both the proximal part (1f) of the catheter head and/or on the remaining catheter section (1e, 1b, 1a).

10. Infusion set according to any one of claims 1 to 6 **characterised in that** the data memory (2) applied on the infusion set is connected via a galvanic connection on the connector to the administration device (3).

11. Infusion set according to claims 1 to 5 **characterised in that** the data memory (2) arranged on the infusion set (1) is connected through an optical transmission method, for example IrDA, with the data exchange device (4) with the administration device (3).

12. Infusion set according to claim 6 **characterised in that** the data memory (2) arranged on the infusion set is connected via a unidirectional optical method, for example a barcode identification method, to the administration device (3).

13. Infusion set according to claim 6 **characterised in that** the data memory arranged on the infusion set is connected via mechanical coding cams on the connector to the administration device (3).

14. System comprising an infusion set for administering a medical fluid according to claim 1 and an administration device (3) envisaged for this.

15. Method for the interaction of an infusion set (1) according to claim 1 with an administration device (3) **characterised in that** if data memory information is available from which it can be deduced that a configuration of an infusion set with an administration unit is present that is not suitable or is not treatment-conforming, a warning message is triggered on the administration device and/or use of the infusion set is prevented.

16. Method according to claim 15 **characterised in that** an automatic or semi-automatic priming procedure controlled by a priming detector is carried out if, in order to provide a treatment-conforming operating status, a priming procedure is required and if data memory information is available in real time which shows the signal status of the priming detector.

17. Method according to claim 15 **characterised in that** if data are available in the data memory (2) which define or make a priming volume of an infusion set determinable, an automatic or semi-automatic priming procedure can be enabled on the administration device (3) .

18. Method according to claim 15 **characterised in that** after exceeding the usage duration of an infusion set of the infusion device (3) connected to an administration device it is invalidated via the data exchange device (4) or identified in the data memory (2) through which reuse is made impossible.

19. Method according to claims 15 and 18 **characterised in that** if data memory information is available from which it can be determined or deduced that an already used or invalidated infusion set has again been connected, the user is requested not to use the infusion set and/or further use is prevented.

20. Method according to claims 15 and 19 **characterised in that** if data memory information is available from which it can be determined or deduced that a connected connection-detector has determined a defective connection between the infusion set and administration device, the user is requested to rectify the fault and use until fault rectification is prevented.

21. Method according to claims 15, 19 and 20 **characterised in that** if data memory information is available from which it can be determined or deduced that a connected occlusion detector has determined an occlusion in the infusion set, the user is requested to eliminate the occlusion.

22. Method according to claims 15, 19, 20 and 21 **characterised in that** if data memory information is available from which it can be determined or deduced that a connected leakage detector has determined leakage in the infusion set, the user is requested to eliminate the cause of the leakage.

## Revendications

1. Ensemble de perfusion pour l'administration d'un liquide médicale, comprenant :
- une mémoire de données (2) pour échange de données par un dispositif d'échange de données (4) avec un dispositif d'administration (3), les informations présentes sur la mémoire de données (2) étant transmises par un dispositif d'échange de données (4) du dispositif d'administration (3) pour constater des écarts par rapport à l'état de fonctionnement conforme à la thérapie, un état de fonctionnement conforme à la thérapie pouvant être de ce fait automatisé ou établi à l'aide d'un support de pompage, la mémoire de données (2) comportant au moins une zone de données écrasable, les données sur les zones de données écrasables pouvant être influencées par le dispositif d'administration autorisé (3),
- au moins un détecteur d'état (5) localisé sur l'ensemble de perfusion, les données sur les zones de données écrasable pouvant être modifiées par un signal initial d'au moins un détecteur d'état (5) localisé sur l'ensemble de perfusion.

2. Ensemble de perfusion selon la revendication 1, **caractérisé en ce que** le détecteur d'état (5) localisé sur l'ensemble de perfusion peut être un capteur d'occlusion, de fuite, de connexion ou d'amorçage.

3. Ensemble de perfusion selon la revendication 2, **caractérisé en ce que** le signal initial d'un détecteur d'état est mémorisé dans les zones de données écrasables sous la forme d'un témoin d'état.

4. Ensemble de perfusion selon la revendication 1, **caractérisé en ce que** des zones de mémoire (WORM) sont présentes sur la mémoire de données (2), lesquelles peuvent être modifiées exactement une fois depuis le dispositif de perfusion par le dispositif d'échange de données pour pouvoir procéder par exemple à une identification ou dévalorisation d'un ensemble de perfusion critique du point de vue sécurité de façon permanente.

5. Ensemble de perfusion selon la revendication 4, **caractérisé en ce que** la mémoire de données WORM (2) est modifiée à expiration de la durée de portée du dispositif de perfusion par le dispositif d'échange de données au sens d'une dévalorisation de telle sorte qu'une nouvelle utilisation est rendue impossible.

6. Ensemble de perfusion selon la revendication 1, **caractérisé en ce que** la mémoire de données (2) comporte au moins une zone de données (ROM) non écrasable, qui contient au moins une caractéristique spécifique au type, laquelle permet de définir ou de déterminer un volume d'amorçage spécifique, afin de pouvoir lancer le cas échéant un processus d'amorçage automatisé ou semi-automatisé de telle sorte qu'il y ait un ensemble de perfusion conforme à la thérapie.

7. Ensemble de perfusion selon l'une quelconque des revendications précédentes, **caractérisé en ce que** le dispositif d'échange de données (4) repose sur la technique de transmission sans fil.

8. Ensemble de perfusion selon la revendication 7, **caractérisé en ce que** le dispositif d'échange de données (4) repose sur la technologie RFID.

9. Ensemble de perfusion selon la revendication 8, **caractérisé en ce qu'**une unité mémoire RFID/transpondeur se trouve respectivement sur la partie proximale (1f) de la tête de cathéter et/ou sur la section de cathéter restante (1e, 1b, 1a).

10. Ensemble de perfusion selon l'une quelconque des revendications 1 à 6, **caractérisé en ce que** la mémoire de données (2) montée sur l'ensemble de perfusion est en liaison avec le dispositif d'administration (3) par une liaison galvanique sur connecteur.

11. Ensemble de perfusion selon l'une quelconque des revendications 1 à 5, **caractérisé en ce que** la mémoire de données (2) montée sur l'ensemble de perfusion (1) est en liaison avec un procédé de transmission optique, comme par exemple IrDA, avec le dispositif d'échange de données (4) avec le dispositif d'administration (3).

12. Ensemble de perfusion selon la revendication 6, **caractérisé en ce que** la mémoire de données (2) montée sur l'ensemble de perfusion est en liaison par un procédé optique unidirectionnel, comme par exemple un procédé d'identification par code à barres, avec le dispositif d'administration (3).

13. Ensemble de perfusion selon la revendication 6, **caractérisé en ce que** la mémoire de données montée sur l'ensemble de perfusion est en liaison avec le dispositif d'administration (3) par un came de codage mécanique sur un connecteur.

14. Système composé d'un ensemble de perfusion pour administration d'un liquide médical selon la revendication 1 et d'un dispositif d'administration (3) prévu à cet effet.

15. Procédé pour l'interaction d'un ensemble de perfusion (1) selon la revendication 1, avec un dispositif d'administration (3) **caractérisé en ce qu'**en présence d'informations de mémoire de données à partir desquelles il est possible de déduire qu'il y a une configuration non adaptée ou non conforme à la thérapie d'un ensemble de perfusion avec un dispositif d'administration, un message d'avertissement est déclenché sur le dispositif d'administration et/ou une utilisation de l'ensemble de perfusion est évitée.

16. Procédé selon la revendication 15, **caractérisé en ce qu'**un processus d'amorçage automatique ou semi-automatique pilotée par un détecteur d'amorçage est effectuée, au cas où un processus d'amorçage est nécessaire pour créer un état de fonctionnement conforme à la thérapie et au cas où des informations de mémoire de données existent en temps réel, lesquelles reproduisent l'état de signal du détecteur d'amorçage.

17. Procédé selon la revendication 15, **caractérisé en ce qu'**en présence de données sur la mémoire de données (2), lesquelles définissent ou rendent déterminables un volume d'amorçage d'un ensemble de perfusion, un processus d'amorçage automatique ou semi-automatique peut être rendu possible sur le dispositif d'administration (3).

18. Procédé selon la revendication 15, **caractérisé en ce qu'**après dépassement de la durée de portée d'un ensemble de perfusion raccordé à un dispositif d'administration, le dispositif de perfusion (3) dévalorise par le dispositif d'échange de données (4) ou identifie sur la mémoire de données (2), une utilisation renouvelée étant de ce fait rendue impossible.

19. Procédé selon la revendication 15 et 18, **caractérisé en ce qu'**en présence d'informations de mémoire de données à partir desquelles il est possible de constater ou de déduire qu'un ensemble de perfusion déjà utilisé ou dévalorisé est à nouveau connecté, il est demandé à l'utilisateur de cesser l'utilisation de l'ensemble de perfusion et/ou d'éviter une utilisation ultérieure.

20. Procédé selon les revendications 15 et 19, **caractérisé en ce qu'**en présence d'informations de mémoire de données à partir desquelles il est possible de constater qu'un détecteur de connexion raccordé constate une connexion défectueuse entre l'ensemble de perfusion et le dispositif d'administration, il est demandé à l'utilisateur d'éliminer le défaut et d'éviter une utilisation avant d'avoir éliminer le défaut.

21. Procédé selon les revendications 15,19 et 20 **caractérisé en ce qu'**en présence d'informations de mémoire de données à partir desquelles il est possible de constater qu'un détecteur d'occlusion raccordé constate une occlusion dans l'ensemble de perfusion, il est demandé à l'utilisateur d'éliminer l'occlusion.

22. Procédé selon les revendications 15, 19, 20 et 21, **caractérisé en ce qu'**en présence d'informations de mémoire de données, à partir desquelles il est possible de constater qu'un détecteur de fuite raccordé constate une fuite dans l'ensemble de perfusion, il est demandé à l'utilisateur d'éliminer les cause de la fuite.
